(19) **Europäisches Patentamt**
**European Patent Office**
**Office européen des brevets**

(11) **EP 3 584 303 B1**

(12) **FASCICULE DE BREVET EUROPEEN**

(45) Date de publication et mention
de la délivrance du brevet:
**17.07.2024  Bulletin 2024/29**

(21) Numéro de dépôt: **19179511.1**

(22) Date de dépôt: **11.06.2019**

(51) Classification Internationale des Brevets (IPC):
**C11D 1/40** *(2006.01)*    **C11D 1/52** *(2006.01)*
**C11D 1/62** *(2006.01)*    **C11D 1/645** *(2006.01)*
**C11D 1/65** *(2006.01)*    **C11D 3/33** *(2006.01)*

(52) Classification Coopérative des Brevets (CPC):
**C11D 1/62; C11D 1/40; C11D 1/52; C11D 1/645;
C11D 1/65; C11D 3/33**

(54) **COMPOSITION TENSIOACTIVE À BASE DE SELS D'AMIDE DE GLYCINE BÉTAÏNE, SON PROCÉDÉ DE PRÉPARATION ET SES UTILISATIONS**

TENSIDZUSAMMENSETZUNG AUF DER BASIS VON AMIDSALZEN VON GLYCINBETAIN, SEIN HERSTELLUNGSVERFAHREN UND SEINE VERWENDUNGEN

SURFACTANT COMPOSITION MADE OF AMIDE SALTS OF GLYCINE BETAINE, METHOD FOR PREPARING SAME AND USES THEREOF

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **19.06.2018  FR 1855359**

(43) Date de publication de la demande:
**25.12.2019  Bulletin 2019/52**

(73) Titulaire: **Surfactgreen
60201 Compiegne Cedex (FR)**

(72) Inventeurs:
• **PESSEL, Freddy
  35137 BEDEE (FR)**
• **GALLE, Francis
  35700 RENNES (FR)**
• **DIVET, Pierre-Yves
  92200 NEUILLY-SUR-SEINE (FR)**
• **ROUSSEL, Xavier
  72000 LE MANS (FR)**

(74) Mandataire: **Renard, Emmanuelle
Renard IP
4 Bis Avenue de Lorraine
92380 Garches (FR)**

(56) Documents cités:
**WO-A1-2005/121291    WO-A1-2013/188508**

Remarques:
Le dossier contient des informations techniques présentées postérieurement au dépôt de la demande et ne figurant pas dans le présent fascicule.

**Description**

OBJET DE L'INVENTION

La présente invention concerne une composition tensioactive à base de sels d'amide de glycine bétaïne, ainsi que son procédé de préparation. Elle porte également sur son utilisation comme agent mouillant, dispersant de particules et/ou inhibiteur de corrosion et/ou pour améliorer le pouvoir désinfectant de substances antimicrobiennes et/ou l'effet de substances insecticides, ainsi que dans la fabrication de différents produits destinés au traitement et/ou au nettoyage du corps, de plantes ou de surfaces dures, au traitement de l'eau ou à l'extraction du pétrole.

ARRIERE-PLAN DE L'INVENTION

Les tensioactifs constituent des matières premières indispensables à la fabrication d'une diversité de produits. Parmi ceux-ci, les tensioactifs cationiques représentent, certes, un marché moins étendu que celui des tensioactifs anioniques ou non ioniques, mais ils présentent toutefois un intérêt dans de multiples applications, notamment dans la fabrication de produits détergents et cosmétiques, ainsi que dans le traitement de l'eau.

En raison de leur toxicité, certains tensioactifs tel que les sels de diméthyldialkylammonium, présents dans la plupart des adoucissants textiles, voient leur utilisation limitée, voire abandonnée, dans certains pays européens comme l'Allemagne et les Pays-Bas. Sous la pression de l'écologie, les producteurs de tensioactifs doivent proposer non seulement des produits moins polluants, plus biodégradables et présentant une écotoxicité la plus faible possible. Aux contraintes environnementales s'ajoute le souhait pour les consommateurs de disposer de produits les plus naturels possibles.

Dans ce contexte, la glycine bétaïne, substance naturelle peu onéreuse, constitue une matière première de choix pour la préparation d'agents tensioactifs. Issue de la mélasse de la betterave à sucre, obtenue après extraction du saccharose, elle reste actuellement un sous-produit de l'industrie sucrière. Le greffage sur la glycine bétaïne d'alcools et d'amines gras permet d'accéder à des molécules amphiphiles cationiques sans l'étape classique de quaternisation d'une amine tertiaire à l'aide d'agents de méthylation généralement toxiques.

Il a ainsi été proposé dans le brevet US-7,829,521 des amides de glycine bétaïne obtenus par réaction de la glycine bétaïne avec un acide sulfonique, tel que l'acide méthanesulfonique, et un alcool, tel que le n-butanol, pour produire un ester de glycine bétaïne qui est ensuite soumis à une réaction d'aminolyse à l'aide d'une amine grasse d'origine végétale comprenant au moins 18 atomes de carbone. Les tensioactifs cationiques ainsi obtenus ont une tension de surface d'au moins 36 mN/m et sont plus particulièrement destinés à une application cosmétique. D'autres tensioactifs similaires à chaîne plus courte sont présentés dans la publication de F. Goursaud et al dans Green Chem., 2008, 10, 310-320, à savoir le laurylamide de glycine bétaïne, dont la tension de surface est, là encore très élevée (50 mN/m). Ce tensioactif est également décrit dans la demande de brevet WO 2013/188508. Dans ce document, il est démontré (tableau 5) que le mélange réactionnel brut présente une tension de surface plus faible (25mN/m) que l'amide de glycine bétaïne pure (38 mN/m), permettant d'envisager son utilisation dans des détergents ménagers. Ce mélange réactionnel renferme précisément 68% de laurylamide, 29% de sels d'ammonium de laurylamine et 3% de glycine bétaïne résiduelle.

En modifiant les conditions opératoires décrites dans WO 2013/188508 les inventeurs ont pu obtenir une composition tensioactive enrichie en amide de glycine bétaïne et appauvrie en sels d'alkylammonium, qui s'est avérée présenter une tension de surface plus faible que les compositions tensioactives décrites dans ce document et convenant de ce fait à de nombreuses autres applications.

Une composition similaire, à base d'un mélange de laurylamide de glycine bétaïne et de myristylamide de lauryl bétaïne a été mentionnée dans le document WO 2017/034793. Toutefois, le procédé très général présenté dans ce document ne permet pas d'obtenir cette composition, *a fortiori* de manière reproductible, faute d'indication sur les paramètres opératoires précis utilisés.

Le procédé de synthèse mis en oeuvre selon l'invention est simple, efficace, respectueux de l'environnement, sans solvant ni rejet polluant, et facilement transposable à l'échelle industrielle pour obtenir de manière reproductible une composition tensioactive particulièrement performante.

RESUME DE L'INVENTION

L'invention a pour objet une composition tensioactive comprenant :

(a) de 70 à 85% en poids d'un ou plusieurs sel(s) d'amide de glycine bétaïne de formule (1) : $X^{n-}[(CH_3)_3N^+-CH_2-CONH-R]_n$ où R est un groupe alkyle linéaire saturé ou insaturé comprenant de 8 à 18 atomes de carbone, étant entendu que 40 à 100% en poids des sels de formule (I) sont constitués de sel de laurylamide de glycine bétaïne ;

(b) de 5 à 20% en poids de sel d'alkylammonium de formule (2) : $X^{n-}[NH_3^+R]_n$ où R est un groupe alkyle linéaire saturé ou insaturé comprenant de 8 à 18 atomes de carbone, étant entendu qu'au moins 40% en poids des sels d'alkylammonium de formule (2) sont constitués de sel de laurylammonium ;

(c) de 5 à 10% en poids de sel d'ester de glycine bétaïne de formule (3) : $X^{n-}[(CH_3)_3\ N^+-CH_2-COOR']_n$ où R' est un radical alkyle linéaire ou ramifié contenant de 4 à 8 atomes de carbone ; et

(d) de 0 à 5% en poids de glycine bétaïne de formule (4) : $(CH_3)_3N^+-CH_2-COO^-$ ;

où :

X est un anion organique ou inorganique, et n vaut 1 ou 2, les composés de formule (1), (2), (3) et (4) représentant au total de 95 à 100% du poids de la composition.

Elle a également pour objet un procédé de préparation de cette composition tensioactive, comprenant les étapes successives consistant à :

(1) faire réagir de la glycine bétaïne ou l'un de ses sels avec un alcool linéaire ou ramifié en $C_4$-$C_8$, en présence d'un acide organique ou inorganique, à une température de 100 à 180°C et sous pression réduite ;
(2) refroidir le milieu réactionnel à une température de 20 à 80°C ;
(3) ajouter une ou plusieurs alkylamines renfermant de 8 à 18 atomes de carbone, dont au moins 40% en poids de laurylamine ;
(4) éliminer l'alcool résiduel ; et
(5) récupérer la composition tensioactive ainsi obtenue,

caractérisé en ce que :

- soit l'étape (1) est mise en oeuvre dans des conditions permettant d'atteindre un taux de conversion de la glycine bétaïne en sel d'ester de glycine bétaïne d'au moins 95%, tel que mesuré par RMN $^1$H et défini par l'équation suivante :

$$\eta = \frac{I_{XOGBOR'}/2}{I_{XOGB}/2 + I_{XOGBOR'}/2} = \frac{I_{XOGBOR'}}{I_{XOGB} + I_{XOGBOR'}}$$

où :
$\eta$ est le taux de conversion

- $I_i$ est la valeur d'intégration du signal caractéristique du composé i [(4,35 ppm, s, 2 H) pour XOGBOR', (4,28 ppm, s, 2 H) pour XOGB quand X = mésylate]
- XOGBOR' désigne le sel de sel de l'ester de glycine bétaïne formé
- XOGB désigne le sel de glycine bétaïne formé,

- soit le sel de l'ester de glycine bétaïne formé dans l'étape (1) est séparé du milieu réactionnel entre les étapes (2) et (3) et mis à réagir avec la ou les alkylamine(s) dans l'étape (4).

L'invention a encore pour objet l'utilisation de la composition tensioactive précitée comme agent mouillant, dispersant de particules et/ou inhibiteur de corrosion et/ou pour améliorer le pouvoir désinfectant et/ou la rémanence de l'effet désinfectant de substances antimicrobiennes et/ou pour améliorer l'effet et/ou la rémanence de substances insecticides.

Elle a également pour objet l'utilisation de cette composition pour la fabrication de plastiques ou de produits destinés :

- au traitement et/ou au nettoyage du corps, de plantes ou de surfaces dures, en particulier de produits cosmétiques, de produits de lavage de véhicules, de produits ménagers, de produits de nettoyage industriel, de produits d'ensimage de fibres et de produits phytosanitaires ;
- au traitement de l'eau ;
- à l'extraction du pétrole.

Outre sa faible tension de surface, la composition tensioactive selon l'invention présente l'avantage d'être biodégradable (selon la norme OECD 310), faiblement toxique pour l'environnement (suivant les normes OECD 201 et 202) et pour l'homme, soluble à froid, stable quel que soit le pH et de présenter un bon pouvoir moussant.

DESCRIPTION DETAILLEE

Composition tensioactive

La composition tensioactive selon l'invention comprend :

(a) de 70 à 85% en poids d'un ou plusieurs sel(s) d'amide de glycine bétaïne de formule (1) : $X^{n-}$[$(CH_3)_3N^+$-$CH_2$-CONH-R]$_n$ où R est un groupe alkyle linéaire saturé ou insaturé comprenant de 8 à 18 atomes de carbone, étant entendu que 40 à 100% en poids des sels de formule (I) sont constitués de sel de laurylamide de glycine bétaïne ;
(b) de 5 à 20% en poids de sel d'alkylammonium de formule (2) : $X^{n-}$[$NH_3^+$R]$_n$ où R est un groupe alkyle linéaire saturé ou insaturé comprenant de 8 à 18 atomes de carbone, étant entendu qu'au moins 40% en poids des sels d'alkylammonium de formule (2) sont constitués de sel de laurylammonium ;
(c) de 5 à 10% en poids de sel d'ester de glycine bétaïne de formule (3) : $X^{n-}$[$(CH_3)_3 N^+$-$CH_2$-COOR']$_n$ où R' est un radical alkyle linéaire ou ramifié contenant de 4 à 8 atomes de carbone ; et
(d) de 0 à 5% en poids de glycine bétaïne de formule (4) : $(CH_3)_3N^+$-$CH_2$-COO$^-$ ;

où :

X est un anion organique ou inorganique
et n vaut 1 ou 2,
où les composés de formule (1), (2), (3) et (4) représentent au total de 95 à 100% du poids de la composition.

Le groupement X peut notamment être choisi parmi les anions issu d'acides organiques ou inorganiques. Il peut s'agir en particulier d'un chlorure, d'un sulfate, d'un perchlorate, d'un ion alkylsulfate, notamment décylsulfate ou laurylsulfate, d'un ion arylsulfonate, notamment benzène sulfonate, paratoluène sulfonate, camphosulfonate, d'un ion alkylsulfonate, notamment triflate, méthanesulfonate, éthanesulfonate, décylsulfonate, laurylsulfonate, d'un ion sulfosuccinate, et de leurs mélanges. On préfère selon l'invention que X soit choisi parmi les alkylsulfonates et les arylsulfonates, en particulier parmi les ions méthanesulfonate, éthanesulfonate, triflate, paratoluènesulfonate et camphosulfonate. Il s'agit avantageusement de l'ion méthanesulfonate.
On préfère par ailleurs que R' désigne le radical butyle, notamment n-butyle.
Avantageusement, la composition selon l'invention comprend exclusivement les composés de formules (1), (2), (3) et (4).

Procédé

La composition tensioactive selon l'invention peut être préparée suivant un procédé tel que décrit précédemment.
La première étape de ce procédé consiste en une réaction d'estérification de la glycine bétaïne, ou triméthylglycine. La glycine bétaïne peut être d'origine végétale ou synthétique. Elle peut éventuellement se présenter sous forme de sel, par exemple de sel inorganique. Il est généralement nécessaire de la protoner préalablement à l'aide d'un acide organique ou inorganique, dans la mesure où elle se présente sous forme zwitterionique (présence d'une fonction carboxylate), sauf dans le cas où on utilise un sel de glycine bétaïne. L'acide peut notamment être choisi parmi les acides inorganiques tels que l'acide chlorhydrique, l'acide sulfurique, les acides perhalohydriques, tel que l'acide perchlorique, et leurs mélanges. En variante, il peut être choisi parmi les acides organiques, tels que les acides alkyl sulfuriques, par exemple l'acide décyl ou lauryl sulfurique ; les acides arylsulfoniques, tels que l'acide benzène sulfonique, l'acide paratoluène sulfonique, l'acide camphosulfonique ; les acides alkylsulfoniques, tels que l'acide triflique, l'acide méthanesulfonique, l'acide éthanesulfonique, l'acide décylsulfonique, l'acide laurylsulfonique ; l'acide sulfosuccinique ; et leurs mélanges.
On peut également utiliser des acides de Lewis. De préférence, il s'agit d'un acide alkylsulfonique et en particulier de l'acide méthanesulfonique.
Au cours de l'estérification proprement dite, l'acide de la bétaïne réagit avec l'alcool linéaire ou ramifié en $C_4$-$C_8$ en présence de l'acide, pour conduire à un ester de glycine bétaïne sous forme de sel. Des exemples d'alcools comprennent le butanol, le pentanol, le 3-méthylbutan-1-ol (ou alcool isoamylique), l'alcool de fusel (mélange de pentanol, de 2-méthylbutan-1-ol et de 3-méthylbutan-1-ol), l'hexanol, l'heptanol, l'octanol et leurs mélanges. Par "butanol", on entend aussi bien dans cette description le n-butanol, l'isobutanol et le sec-butanol. Le butanol, et plus particulièrement le n-butanol, est préféré pour une utilisation dans cette invention. Cette réaction s'effectue généralement en l'absence de tout solvant, l'alcool utilisé constituant à la fois le réactif et le milieu. L'eau produite lors de la réaction contribue également à la solubilisation de la glycine bétaïne dans le mélange réactionnel.

Pour la mise en oeuvre de cette étape, on peut utiliser de 1,1 à 20 équivalents, par exemple de 2 à 4 équivalents, d'alcool linéaire ou ramifié en $C_4$-$C_8$ et de 1,0 à 1,5 équivalents d'acide sulfonique, par exemple de 1,0 à 1,2 équivalent et préférentiellement 1,1 équivalent d'acide sulfonique, pour 1 équivalent de glycine bétaïne. L'estérification peut être réalisée à une température de 100 à 180°C, préférentiellement de 100 à 160°C, plus préférentiellement de 120 à 150°C ou de 130 à 160°C sous pression atmosphérique ou sous pression réduite. Au cours de cette réaction, l'équilibre est déplacé vers la formation du produit de la réaction par distillation du mélange eau-alcool, typiquement à l'aide d'un montage Dean-Stark.

Dans une première variante de l'invention, les conditions de l'estérification sont ajustées pour atteindre un taux de conversion d'au moins 95%. Pour ce faire, le milieu réactionnel est placé sous pression réduite s'il ne l'était pas déjà. La pression sera généralement d'autant plus faible que la longueur de chaîne de l'alcool gras mis en jeu est grande. L'homme de l'art saura en tout état de cause ajuster la pression choisie de manière à éliminer l'eau formée lors de la réaction et à déplacer l'équilibre vers la formation de l'ester. De même, la durée de la réaction sera ajustée en fonction de l'alcool utilisé. Dans le cas du butanol, la distillation peut par exemple être débutée après 2 à 4h de temps de réaction et poursuivie pendant 2 à 4h avant d'abaisser la pression, par exemple à une valeur de 500 à 900 mbar, notamment de 600 à 800 mbar.

Contrairement aux procédés de synthèse d'amides de glycine bétaïne connus, le taux de conversion de la glycine bétaïne en sel de sulfonate d'ester de glycine bétaïne est suivi par RMN $^1$H et la réaction est poursuivie jusqu'à ce qu'il atteigne une valeur d'au moins 95%, c'est-à-dire pendant une durée allant par exemple de 5 à 48h.

Une fois cette étape terminée, et contrairement aux procédés de l'art antérieur, il n'est pas utile d'ajouter au milieu réactionnel une base organique forte et encombrée, telle que la dibutylamine.

Le milieu réactionnel est ensuite refroidi à une température de 20 à 80°C, de préférence de 40 à 80°C dans la première variante décrite ci-dessus.

Dans une seconde variante du procédé selon l'invention, il n'est pas nécessaire que le taux de conversion de la glycine bétaïne soit d'au moins 95% et il peut être de 75 à 90% seulement, par exemple d'environ 80%, avant l'étape de refroidissement précitée, qui s'effectuera dans ce cas de préférence jusqu'à une valeur de 20 à 40°C. Dans cette seconde variante, le produit de la réaction d'estérification est toutefois traité de manière à séparer le sel de l'ester de glycine bétaïne formé du milieu réactionnel. Pour ce faire, on peut par exemple procéder à une filtration du milieu réactionnel, qui permet de séparer l'ester salifié précité, soluble dans l'alcool, des autres constituants qui ne sont pas solubles.

On ajoute ensuite soit au milieu réactionnel (première variante), soit à l'ester isolé (seconde variante) une ou plusieurs alkylamine(s) en C8-C16 dont au moins 40% en poids de laurylamine. Pour ce faire, on peut utiliser soit de la laurylamine seule, soit un mélange de laurylamine avec d'autres alkylamines, obtenu par exemple à partir d'huile de coco. Un tel mélange renferme typiquement de 40 à 60% en poids de laurylamine, de 15 à 22% en poids de myristylamine, de 5 à 12% en poids de palmitylamine, de 2 à 12% en poids de stéarylamine, de 4 à 7% en poids de caprylamine et de 3 à 7% en poids de caprylylamine. L'ajout d'alkylamine(s) est de préférence effectué après avoir éliminé une partie de l'alcool résiduel et les traces d'eau résiduelles par distillation sous pression réduite. Dans cette étape, l'alkylamine est avantageusement utilisée sous forme fondue. La quantité d'alkylamine(s) ajoutée peut par exemple représenter de 0,9 à 1,5 équivalent et de préférence de 1,0 à 1,2 équivalent pour 1 équivalent de glycine bétaïne initialement mise en oeuvre. Cette réaction d'aminolyse est typiquement réalisée à une température de 50 à 180°C et de préférence de 120 à 140°C, sous pression réduite, par exemple sous une pression de 1 à 30 mbar.

En parallèle de la réaction d'aminolyse, l'alcool est éliminé par distillation sous pression réduite. La réaction d'aminolyse et la distillation se font pendant une durée de 1 à 7 heures, notamment de 3 à 5 heures.

On récupère alors la composition tensioactive ainsi obtenue.

Utilisations

La composition tensioactive selon l'invention présente une valeur de tension de surface inférieure à 24 mN/m, voire inférieure à 22 mN/m et généralement supérieure ou égale à 20 mN/m, mesurée selon la norme NF EN 14370.

Il est ainsi possible d'envisager son utilisation dans une diversité d'applications comme agent mouillant, dispersant de particules et/ou inhibiteur de corrosion et/ou pour améliorer le pouvoir désinfectant de substances antimicrobiennes et/ou l'effet de substances insecticides. Elle peut en particulier être utilisée pour la fabrication de plastiques ou de différents produits destinés notamment :

- au traitement et/ou au nettoyage du corps, de plantes, de textiles ou de surfaces dures, en particulier de produits cosmétiques, tels que des shampooings, savons liquides, bains moussants et gels-douches ; de produits de lavage de véhicules tels que des automobiles, des camions, des trains, des bus ou des avions ; de produits ménagers tels que des détergents pour les vitres, les surfaces murales, les sols ou la vaisselle ; de lessives ou d'adoucissants pour le linge ; de produits de nettoyage industriel ; de produits d'ensimage de fibres ; de produits phytosanitaires ; de produits pigmentés tels que des peintures ou vernis ;

- au traitement de l'eau ;
- à l'extraction du pétrole.

Dans le cas où elle est utilisée dans le nettoyage de surfaces dures, tels que des vitres ou des surfaces de carrosserie, ou encore de textiles, il a en particulier été observé que la composition selon l'invention accélérait le séchage ultérieur de la surface sans laisser subsister de traces de calcaire au séchage. En outre, lorsque la surface est un véhicule, il a été observé que le nettoyage des particules fines de frein sur les roues était amélioré par rapport à des tensioactifs cationiques classiques. Enfin, l'efficacité de la composition selon l'invention en milieu alcalin permet d'éviter les inconvénients liés à l'emploi de compositions acides, en particulier leur effet corrosif.

Dans le cas du traitement de l'eau, la composition selon l'invention permet de décoller le biofilm sans détruire l'efficacité des résines échangeuses d'ions, contrairement aux tensioactifs cationiques classiques qui présentent par ailleurs un impact environnemental non négligeable compte tenu de leur absence de biodégradabilité, ou de leur biodégradabilité plus lente. Cette capacité à décoller les biofilms peut également être mise à profit dans les procédés d'extraction de pétrole.

Dans des applications cosmétiques, la composition selon l'invention est compatible avec les tensioactifs anioniques classiques et permet d'améliorer le caractère crémeux de la mousse qu'ils génèrent. Elle protège également les dispositifs aérosols en fer contre la corrosion.

Dans la fabrication de plastiques, la composition selon l'invention permet de conférer des propriétés électrostatiques à la surface du plastique, sans affecter ses capacités de recyclage compte tenu de son caractère biosourcé.

Lorsqu'elle est utilisée dans la fabrication de produits phytosanitaires, la composition selon l'invention permet d'améliorer la rémanence des matières actives et la résistance à l'eau des produits tels que des herbicides, des pesticides ou des agents modifiant la croissance des plantes, qui peuvent ainsi être utilisés en plus faible quantité. Cette composition peut ainsi être ajoutée, sous forme diluée à 25% dans l'eau, à raison de 0,4% en poids, dans un produit renfermant un milieu neutre ou alcalin, par exemple.

La composition selon l'invention peut par ailleurs être utilisée dans un procédé d'extraction, de stockage, d'entreposage ou de raffinage de pétrole pour limiter la corrosion des équipements. Dans cette application, elle peut être ajoutée au pétrole à hauteur de 500 à 1000 ppm, par exemple.

La composition selon l'invention peut être incluse dans un produit comprenant au moins un composé choisi parmi : les tensioactifs anioniques, les tensioactifs non ioniques, les agents anti-microbiens et/ou les substances insecticides et leurs mélanges. Des exemples de tensioactifs anioniques sont : les sels de sulfate d'alcools gras éthoxylés, les sulfosuccinates, les sarcosinates, les alkyl- et dialkylphosphates, les savons d'acides gras et leurs mélanges. Les tensioactifs non ioniques peuvent par exemple être choisis parmi : les esters d'acides gras et de polyols tel que les esters d'acides gras et de glycérol éventuellement polyéthoxylés, les esters d'acides gras et de sorbitane éventuellement polyéthoxylés, les esters d'acides gras et de polyoxyéthylène, les esters d'acides gras et de sucrose comme le stéarate de sucrose; les éthers d'alcool gras et de polyoxyéthylène, les éthers d'alcools gras et de sucre, notamment les alkylpolyglucosides (APG), les polysiloxanes modifiés polyéthers, et leurs mélanges. Les agents anti-microbiens peuvent être choisis parmi les ammoniums quaternaires, les aldéhydes (tels que le glutaraldéhyde et le formaldéhyde), l'éthanol, les dérivés halogénés, les oxydants, les composés phénoliques, les parabens, les isothiazolones (ou isothiazolinones), les benzoates, l'imidazoline, l'hydantoïne, la guanidine, les acides organiques tels que l'acide lactique, et leurs mélanges. Les substances insecticides peuvent être choisies parmi les agents organosphosphorés (tels que l'acéphate, le chlorpyrifos ou le bromophos), les nicotinoïdes, les pyréthrinoïdes (tels que la perméthrine, la bifenthrine ou la fenvalérate), les monoterpènes (tels que le p-menthane-3,8-diol), les composés organohalogénés (tels que le lindane, le dicofol ou le toxaphène), le N,N-diéthyl-3-méthylbenzamide, les dérivés du pyrèthre (tels que la Pyréthrine I, la Pyréthrine II ou la Jasmoline I), les sulfones, les sulfonates, les formamidines, les benzoylurées, les roténones, les alcaloïdes, la quassine, la ryanidone, l'aconitine, le géraniol et leurs mélanges.

Ce produit se présente avantageusement sous forme de solution aqueuse ou de gel aqueux. En variante, il peut se présenter sous forme d'émulsion huile-dans-eau ou eau-dans-huile voire de pâte. En tout état de cause, la phase aqueuse contenue dans ce produit présente avantageusement un pH allant de 1 à 12, notamment de 8 à 12 et de préférence de 9 à 11. Ce produit peut être conditionné dans tout dispositif adapté à l'utilisation envisagée et notamment dans un flacon-pompe, un tube, un pot, un dispositif aérosol ou une lingette.

Il renferme avantageusement de 0,1 à 25% en poids, par exemple de 1 à 10% en poids, de composition tensioactive selon l'invention.

Le produit précité peut également comprendre, en plus des agents anti-microbiens, des substances insecticides et des tensioactifs précités, et suivant l'application envisagée, au moins un ingrédient choisi parmi : les actifs phytosanitaires ou cosmétiques, les enzymes, les agents chélatants, les épaississants, les corps gras (huiles, cires et/ou pâteux), les charges, les conservateurs, les pigments et colorants, les anti-oxydants, les azurants optiques, et leurs mélanges.

EXEMPLES

L'invention sera mieux comprise à la lumière des exemples suivants, qui sont donnés à titre purement illustratif et n'ont pas pour but de limiter la portée de l'invention, définie par les revendications annexées.

**Exemple 1** : **Synthèse d'une composition tensioactive selon l'invention (première variante)**

La glycine bétaïne (1,0 éq), le butanol (3,0 éq) et la solution d'acide méthanesulfonique 70 % (1,1 éq) sont introduits dans un réacteur surmonté d'un réfrigérant. Le mélange est chauffé à 140°C sous pression atmosphérique. A 3 h de réaction, un Dean-Stark rempli de butanol est monté sur le réacteur. Le mélange est laissé sous pression atmosphérique car la distillation de l'azéotrope eau-butanol est au départ suffisamment importante. Après 3 h de réaction supplémentaires, lorsque la vitesse de distillation de l'azéotrope eau-butanol diminue, la pression est réduite jusqu'à 700 mbar afin d'accélérer l'élimination de l'eau et permettre le déplacement de l'équilibre vers l'ester butylique de glycine bétaïne. Le taux de conversion est suivi par des analyses RMN $^1$H.

- La méthode par RMN consiste à réaliser un spectre $^1$H de l'échantillon dissout dans un mélange CDCl$_3$/CD$_3$OD (1/1, v /v) en prenant le signal du méthanol comme référence à 3,31 ppm. Les signaux caractéristiques des différents composés sont ensuite intégrés : MsOGBOBu ( 4,35 ppm, s, 2 H), MsOGB (4,28 ppm, s, 2 H), butanol (3,53 ppm, t, 2 H), méthanesulfonate (2,74 ppm, s, 3 H), éther dibutylique (3,40 ppm, t, 4 H), où XOGBOBu désigne le sel de sulfonate de l'ester de glycine bétaïne formé et XOGB désigne le sulfonate de glycine bétaïne formé. Le signal caractéristique du méthanesulfonate tient compte à la fois de l'acide méthanesulfonique présent dans le milieu, mais également du méthanesulfonate qui est le contre ion de la glycine bétaïne et du mésylate de bétaïnate de butyle (MsOGBOBu).

Le taux de conversion de la réaction est obtenu grâce aux valeurs d'intégration par le calcul suivant :

$$\eta = \frac{I_{MsOGBOBu}/2}{I_{MsOGB}/2 + I_{MSOGBOBu}/2} = \frac{I_{MsOGBOBu}}{I_{MsOGB} + I_{MsOGBOBU}}$$

où :

- $\eta$ est le taux de conversion
- $I_i$ est la valeur d'intégration du signal caractéristique du composé i.

Une fois que le taux de conversion de la réaction d'estérification atteint 98 %, le mélange réactionnel est laissé refroidir jusqu' à 60°C. Pendant cette phase de refroidissement, le montage Dean-Stark est remplacé par un montage de distillation et le réacteur est placé sous pression réduite afin d'éliminer une partie du butanol et les traces d'eau restantes dans le mélange réactionnel. Une fois le mélange à 60°C, la laurylamine (1,1 éq) préalablement fondue est ajoutée. Le mélange réactionnel est alors chauffé à 130°C sous pression réduite. La pression est progressivement diminuée jusqu'à 10 mbar. Après la distillation totale du butanol (environ 4 heures), le mélange réactionnel est récupéré et constitue la composition tensioactive.

Cette dernière présente la composition massique suivante :

| Constituant | Masse molaire (g/mol) | % en poids |
|---|---|---|
| Mésylate de bétaïnylaminododécane | 380,588 | 77% |
| Mésylate de dodécylammonium | 281,455 | 15% |
| Mésylate de bétaïnate de butyle | 269,356 | 7% |
| Glycine bétaïne | 117,148 | 1% |
| Butanol | 74,120 | 0% |

On a mesuré la tension de surface de cette composition tensioactive à la CMC, après ajustement du pH à 10 à l'aide de soude.

La mesure de tension superficielle a été réalisée selon la norme NF EN 14370, à l'aide d'un tensiomètre Krüss avec un anneau en platine suspendu horizontalement. Avant chaque mesure, l'anneau est minutieusement nettoyé et séché à la flamme. Le godet pour échantillon est un récipient conique en PTFE placé dans une enceinte thermorégulée à 25°C. L'échantillon est préparé avec de l'eau Milli-Q et continuellement agité à l'aide d'un barreau magnétique avant chaque mesure.

La tension de surface ainsi mesurée était de 22 mN/m.

**Exemple 2 : Synthèse d'une composition tensioactive selon l'invention (seconde variante)**

La glycine bétaïne (1,0 éq) et le butanol (3,0 éq) sont introduits dans un réacteur surmonté d'un Dean-Starck rempli de butanol. Le mélange est chauffé à 140°C sous une pression réduite de 700 mbar. Une fois que les consignes de température et de pression sont atteintes, la solution d'acide méthanesulfonique 70 % est ajoutée au mélange. Le taux de conversion est suivi par des analyses RMN [1]H. Une fois que le taux de conversion de la réaction d'estérification atteint environ 80 %, le mélange réactionnel est laissé revenir à température ambiante et pression atmosphérique. Le mélange est ensuite filtré afin de séparer le mésylate de glycine bétaïne solide du mésylate de bétaïnate de butyle qui est solubilisé dans le butanol. Le filtrat est introduit dans un réacteur surmonté d'un montage de distillation. La laurylamine (0,9-1,5 éq) préalablement fondue est ajoutée. Le mélange réactionnel est alors chauffé à 130°C sous pression réduite. La pression est progressivement diminuée jusqu'à 10 mbar. Après la distillation totale du butanol (environ 4 heures), le mélange réactionnel est récupéré et constitue la composition tensioactive selon l'invention.

**Exemple 3 : Synthèse d'une composition tensioactive comparative**

On a préparé une composition tensioactive de manière similaire au procédé décrit à l'exemple 1, excepté que les conditions d'estérification ont été ajustées de manière à obtenir un taux de conversion de 91% seulement.

Pour ce faire, la glycine bétaïne (1,0 éq), le butanol (3,0 éq) et la solution d'acide méthanesulfonique 70 % (1,1 éq) sont introduits dans un réacteur surmonté d'un Dean-Stark rempli de butanol. Le mélange est chauffé à 140°C sous pression atmosphérique. Malgré un temps de réaction prolongé, le taux de conversion ne dépasse pas 91 %.

Le mélange réactionnel est laissé refroidir jusqu'à 60°C. Une fois le mélange à 60°C, la laurylamine (1,1 éq) préalablement fondue est ajoutée. Le mélange réactionnel est alors chauffé à 130°C sous pression réduite. La pression est progressivement diminuée jusqu'à 10 mbar. Après la distillation totale du butanol (environ 4 heures), le mélange réactionnel est récupéré et constitue la composition tensioactive.

La composition massique du tensioactif ainsi obtenu est la suivante :

| Constituant | Masse molaire (g/mol) | % en poids |
|---|---|---|
| Mésylate de bétaïnylaminododécane | 380,588 | 68% |
| Mésylate de dodécylammonium | 281,455 | 23% |
| Mésylate de bétaïnate de butyle | 269,356 | 6% |
| Glycine bétaïne | 117,148 | 3% |
| Butanol | 74,120 | 0% |

Cette composition renferme donc moins de laurylamide de glycine bétaïne et plus de sel de laurylammonium que la composition selon l'invention. Cette différence a un impact direct sur la tension de surface de cette composition, qui s'avère être plus élevée que celle de la composition tensioactive selon l'invention (25 mN/m à la CMC).

**Exemple 4 : Synthèse d'une composition tensioactive selon l'invention**

La glycine bétaïne (1,0 éq) et l'hexanol (3,0 éq) sont introduits dans un réacteur surmonté d'un Dean-Stark rempli d'hexanol. Sur le couvercle du réacteur est fixée une ampoule de coulée isobare contenant une solution d'acide méthanesulfonique 70 % (1,1 éq). Le mélange est agité et chauffé à 160°C sous pression réduite à 650 mbar. Une fois les conditions réactionnelles atteintes, la solution d'acide méthanesulfonique 70 % est introduite progressivement au mélange réactionnel. Une fois l'ajout terminé, la pression est diminuée régulièrement jusqu'à atteindre 350 mbar afin d'accélérer l'élimination de l'eau et permettre le déplacement de l'équilibre vers l'ester de glycine bétaïne. Le taux de conversion est suivi par des analyses RMN 1H.

La méthode par RMN consiste à réaliser un spectre $^1$H de l'échantillon dissout dans un mélange $CDCl_3/CD_3OD$

(1/1, v /v) en prenant le signal du méthanol comme référence à 3,31 ppm. Les signaux caractéristiques des différents composés sont ensuite intégrés : MsOGBOC6 ( 4,35 ppm, s, 2 H), MsOGB (4,28 ppm, s, 2 H), hexanol (3,53 ppm, t, 2 H), méthanesulfonate (2,74 ppm, s, 3 H), éther dihexylique (3,40 ppm, t, 4 H), où XOGBOC6 désigne le sel de sulfonate de l'ester de glycine bétaïne formé et XOGB désigne le sulfonate de glycine bétaïne formé. Le signal caractéristique du méthanesulfonate tient compte à la fois de l'acide méthanesulfonique présent dans le milieu, mais également du méthanesulfonate qui est le contre-ion de la glycine bétaïne et du mésylate de bétaïnate d'hexyle (MsOGBOC6).

Le taux de conversion de la réaction est obtenu grâce aux valeurs d'intégration par le calcul suivant :

$$\eta = \frac{I_{MsOGBOC6}/2}{I_{MsOGB}/2 + I_{MsOGBOC6}/2} = \frac{I_{MsOGBOC6}}{I_{MsOGB} + I_{MsOGBOC6}}$$

où :

- $\eta$ est le taux de conversion
- $I_i$ est la valeur d'intégration du signal caractéristique du composé i.

Une fois que le taux de conversion de la réaction d'estérification atteint 98 %, le mélange réactionnel est laissé refroidir jusqu' à 60°C. Pendant cette phase de refroidissement, le montage Dean-Stark est remplacé par un montage de distillation et le réacteur est placé sous pression réduite afin d'éliminer une partie de l'hexanol et les traces d'eau restantes dans le mélange réactionnel. Une fois le mélange à 60°C, la laurylamine (1,1 éq) préalablement fondue est ajoutée. Le mélange réactionnel est alors chauffé à 150°C sous pression réduite. La pression est progressivement diminuée jusqu'à 10 mbar. Après la distillation totale de l'hexanol (environ 4 heures), le mélange réactionnel est récupéré et constitue la composition tensioactive.

Cette dernière présente la composition massique suivante :

| Constituant | Masse molaire (g/mol) | % en poids |
|---|---|---|
| Mésylate de bétaïnylaminododécane | 380,588 | 76% |
| Mésylate de dodécylammonium | 281,455 | 14% |
| Mésylate de bétaïnate d'hexyle | 297,41 | 9% |
| Glycine bétaïne | 117,148 | 1% |
| Hexanol | 102,177 | 0% |

On a mesuré la tension de surface de cette composition tensioactive à la CMC, après ajustement du pH à 10 à l'aide de soude.

La mesure de tension superficielle a été réalisée selon la norme NF EN 14370, à l'aide d'un tensiomètre Krüss avec un anneau en platine suspendu horizontalement. Avant chaque mesure, l'anneau est minutieusement nettoyé et séché à la flamme. Le godet pour échantillon est un récipient conique en PTFE placé dans une enceinte thermorégulée à 25°C. L'échantillon est préparé avec de l'eau Milli-Q et continuellement agité à l'aide d'un barreau magnétique avant chaque mesure.

La tension de surface ainsi mesurée était de 20 mN/m.

**Exemple 5** : **Synthèse d'une composition tensioactive selon l'invention**

La glycine bétaïne (1,0 éq) et l'octanol (3,0 éq) sont introduits dans un réacteur surmonté d'un Dean-Stark rempli d'octanol. Sur le couvercle du réacteur est fixée une ampoule de coulée isobare contenant une solution d'acide méthanesulfonique 70 % (1,1 éq). Le mélange est agité et chauffé à 160°C sous pression réduite à 650 mbar. Une fois les conditions réactionnelles atteintes, la solution d'acide méthanesulfonique 70 % est introduite progressivement au mélange réactionnel. Une fois l'ajout terminé, la pression est diminuée régulièrement jusqu'à atteindre 100 mbar afin d'accélérer l'élimination de l'eau et permettre le déplacement de l'équilibre vers l'ester de glycine bétaïne. Le taux de conversion est suivi par des analyses RMN 1H.

- La méthode par RMN consiste à réaliser un spectre 1H de l'échantillon dissout dans un mélange CDCl$_3$/CD$_3$OD (1/1, v /v) en prenant le signal du méthanol comme référence à 3,31 ppm. Les signaux caractéristiques des différents composés sont ensuite intégrés : MsOGBOC8 (4,35 ppm, s, 2 H), MsOGB (4,28 ppm, s, 2 H), octanol (3,53 ppm, t, 2 H), méthanesulfonate (2,74 ppm, s, 3 H), éther dioctylique (3,40 ppm, t, 4 H), où XOGBOC8 désigne le sel de sulfonate de l'ester de glycine bétaïne formé et XOGB désigne le sulfonate de glycine bétaïne formé. Le signal caractéristique du méthanesulfonate tient compte à la fois de l'acide méthanesulfonique présent dans le milieu, mais également du méthanesulfonate qui est le contre ion de la glycine bétaïne et du mésylate de bétaïnate d'octyle (MsOGBOC8).

Le taux de conversion de la réaction est obtenu grâce aux valeurs d'intégration par le calcul suivant :

$$\eta = \frac{I_{MsOGBOC8}/2}{I_{MsOGB}/2 + I_{MsOGBOC8}/2} = \frac{I_{MsOGBOC8}}{I_{MsOGB} + I_{MsOGBOC8}}$$

où :

- $\eta$ est le taux de conversion
- $I_i$ est la valeur d'intégration du signal caractéristique du composé i.

Une fois que le taux de conversion de la réaction d'estérification atteint 99 %, le mélange réactionnel est laissé refroidir jusqu' à 60°C. Pendant cette phase de refroidissement, le montage Dean-Stark est remplacé par un montage de distillation et le réacteur est placé sous pression réduite afin d'éliminer une partie de l'octanol et les traces d'eau restantes dans le mélange réactionnel. Une fois le mélange à 60°C, la laurylamine (1,1 éq) préalablement fondue est ajoutée. Le mélange réactionnel est alors chauffé à 150°C sous pression réduite. La pression est progressivement diminuée jusqu'à 5 mbar. Après la distillation totale de l'octanol (environ 4 heures), le mélange réactionnel est récupéré et constitue la composition tensioactive.

Cette dernière présente la composition massique suivante :

| Constituant | Masse molaire (g/mol) | % en poids |
|---|---|---|
| Mésylate de bétaïnylaminododécane | 380,588 | 75% |
| Mésylate de dodécylammonium | 281,455 | 13% |
| Mésylate de bétaïnate d'octyle | 297,41 | 10% |
| Glycine bétaïne | 117,148 | 1% |
| Octanol | 102,177 | 1% |

On a mesuré la tension de surface de cette composition tensioactive à la CMC, après ajustement du pH à 10 à l'aide de soude.

La mesure de tension superficielle a été réalisée selon la norme NF EN 14370, à l'aide d'un tensiomètre Krüss avec un anneau en platine suspendu horizontalement. Avant chaque mesure, l'anneau est minutieusement nettoyé et séché à la flamme. Le godet pour échantillon est un récipient conique en PTFE placé dans une enceinte thermorégulée à 25°C. L'échantillon est préparé avec de l'eau Milli-Q et continuellement agité à l'aide d'un barreau magnétique avant chaque mesure.

La tension de surface ainsi mesurée était de 20 mN/m.

**Exemple 6 : Formulations**

Plusieurs types de produits peuvent être préparés à l'aide de la composition tensioactive selon l'invention, désignée ci-après par "GBA C12".

Détergent ménager

| | |
|---|---|
| Acide lactique 80% | 2,00 % |
| GBA C12 | 0,40 % |

(suite)

| Hydroxyéthyl cellulose | | 0,30 % |
|---|---|---|
| Chélatant | | 0,20 % |
| Parfum | | 0,20 % |
| Colorant | | 0,01 % |
| Eau désionisée | qsp | 100,00 % |

Ce produit peut être utilisé pour le nettoyage de surfaces dures.

Shampooing carrosserie

| GBA C12 | | 3-5% |
|---|---|---|
| Alcool éthoxylés | | 0-5% |
| Agent chélatant* | | 5-10% |
| Soude | | 0,5-2% |
| Eau | qsp | 100% |
| * Dissolvine® GL d'AKZO NOBEL ou Trilon® M de BASF | | |

Ce produit peut être appliqué sur un véhicule puis, après un temps de pose de 5 minutes, être rincé sous haute pression.

Traitement de l'eau

| MEA (monoethanol amine) | 5-10% |
|---|---|
| GBA C12 | 20-25% |
| Polymère antiredéposition | 10-25% |
| Eau | qsp 100% |

Insecticide

| Pyrèthre | 2% |
|---|---|
| Solubilisant | 3% |
| Ester de sorbitane | 2% |
| GBA C12 | 1% |
| Phéromone | 0,2% |
| Agent antimousse | 0,1% |
| Eau | qsp 100 % |

## Revendications

1. Composition tensioactive comprenant :

(a) de 70 à 85% en poids d'un ou plusieurs sel(s) d'amide de glycine bétaïne de formule (1) : $X^{n-}[(CH_3)_3N^+\text{-}CH_2\text{-}CONH\text{-}R]_n$ où R est un groupe alkyle linéaire saturé ou insaturé comprenant de 8 à 18 atomes de carbone, étant entendu que 40 à 100% en poids des sels de formule (I) sont constitués de sel de laurylamide de glycine bétaïne ;
(b) de 5 à 20% en poids de sel d'alkylammonium de formule (2) : $X^{n-}[NH_3^+R]_n$ où R est un groupe alkyle linéaire saturé ou insaturé comprenant de 8 à 18 atomes de carbone, étant entendu qu'au moins 40% en poids des sels d'alkylammonium de formule (2) sont constitués de sel de laurylammonium ;

(c) de 5 à 10% en poids de sel d'ester de glycine bétaïne de formule (3) : $X^n\text{-}[(CH_3)_3 N^+\text{-}CH_2\text{-}COOR']_n$ où R' est un radical alkyle linéaire ou ramifié contenant de 4 à 8 atomes de carbone ; et

(d) de 0 à 5% en poids de glycine bétaïne de formule (4) : $(CH_3)_3N^+\text{-}CH_2\text{-}COO^-$ ;

où :

X est un anion organique ou inorganique
et n vaut 1 ou 2,
les composés de formule (1), (2), (3) et (4) représentant au total de 95 à 100% du poids de la composition.

2. Composition selon la revendication 1, **caractérisée en ce que** le groupement X est choisi parmi les anions issus d'acides organiques ou inorganiques, constitués d'un chlorure, d'un sulfate, d'un perchlorate, d'un ion alkylsulfate, notamment décylsulfate ou laurylsulfate, d'un ion arylsulfonate, notamment benzène sulfonate, paratoluène sulfonate, camphosulfonate, d'un ion alkylsulfonate, notamment triflate, méthanesulfonate, éthanesulfonate, décylsulfonate, laurylsulfonate, d'un ion sulfosuccinate, et de leurs mélanges, de préférence X est choisi parmi les alkylsulfonates et les arylsulfonates, en particulier parmi les ions méthanesulfonate, éthanesulfonate, triflate, paratoluènesulfonate et camphosulfonate, plus préférentiellement X est l'ion méthanesulfonate.

3. Composition selon la revendication 1 ou 2, **caractérisée en ce que** la composition comprend exclusivement les composés de formules (1), (2), (3) et (4).

4. Procédé de préparation de la composition selon l'une quelconque des revendications 1 à 3, comprenant les étapes successives consistant à :

1) faire réagir de la glycine bétaïne ou l'un de ses sels avec un alcool linéaire ou ramifié en $C_4\text{-}C_8$, en présence d'un acide organique ou inorganique, à une température de 100 à 180°C et sous pression réduite;
2) refroidir le milieu réactionnel à une température de 20 à 80°C ;
3) ajouter une ou plusieurs alkylamines renfermant de 8 à 18 atomes de carbone, dont au moins 40% en poids de laurylamine ;
4) éliminer l'alcool résiduel ; et
5) récupérer la composition tensioactive ainsi obtenue,

**caractérisé en ce que** :

- soit l'étape (1) est mise en oeuvre dans des conditions permettant d'atteindre un taux de conversion de la glycine bétaïne en sel d'ester de glycine bétaïne d'au moins 95%, tel que mesuré par RMN $^1$H et défini par l'équation suivante :

$$\eta = \frac{I_{XOGBOR'}/2}{I_{XOGB}/2 + I_{XOGBOR'}/2} = \frac{I_{XOGBOR'}}{I_{XOGB} + I_{XOG}}$$

où :

• $\eta$ est le taux de conversion

• $I_i$ est la valeur d'intégration du signal caractéristique du composé i [(4,35 ppm, s, 2 H) pour XOGBOR', (4,28 ppm, s, 2 H) pour XOGB quand X = mésylate]
• XOGBOR' désigne le sel de l'ester de glycine bétaïne formé
• XOGB désigne le sel de glycine bétaïne formé,

- soit le sel de l'ester de glycine bétaïne formé dans l'étape (1) est séparé du milieu réactionnel entre les étapes (2) et (3) et mis à réagir avec la ou les alkylamine(s) dans l'étape (4).

5. Procédé selon la revendication 4, **caractérisé en ce que** l'alcool est choisi parmi : le butanol, le pentanol, le 3-méthylbutan-1-ol (ou alcool isoamylique), l'alcool de fusel (mélange de pentanol, de 2-méthylbutan-1-ol et de 3-méthylbutan-1-ol), l'hexanol, l'heptanol, l'octanol et leurs mélanges, de préférence le butanol, plus préférentiellement le n-butanol.

6. Procédé selon la revendication 4 ou 5, **caractérisé en ce qu'**on utilise de 1,1 à 20 équivalents d'alcool, de 1,0 à 1,5 équivalents d'acide sulfonique et de 0,9 à 1,5 équivalent d'alkylamine, pour 1 équivalent de glycine bétaïne.

7. Utilisation de la composition selon l'une quelconque des revendications 1 à 3 comme agent mouillant, dispersant de particules et/ou inhibiteur de corrosion et/ou pour améliorer le pouvoir désinfectant et/ou la rémanence de l'effet désinfectant de substances antimicrobiennes et/ou l'effet de substances insecticides.

8. Utilisation de la composition selon l'une quelconque des revendications 1 à 3 pour la fabrication de plastiques ou de produits destinés :

- au traitement et/ou au nettoyage du corps, de plantes ou de surfaces dures, en particulier de produits cosmétiques, de produits de lavage de véhicules, de produits ménagers, de produits de nettoyage industriel, de produits d'ensimage de fibres et de produits phytosanitaires ;
- au traitement de l'eau ;
- à l'extraction du pétrole.

**Patentansprüche**

1. Tensidzusammensetzung, die Folgendes umfasst:

(a) 70 bis 85 Gew.-% eines oder mehrerer Glycinbetainamidsalze der Formel (1) : $X^{n-}[(CH_3)_3N^+-CH_2-CONH-R]_n$, wobei R eine gesättigte oder ungesättigte lineare Alkylgruppe ist, die 8 bis 18 Kohlenstoffatome umfasst, mit der Maßgabe, dass 40 bis 100 Gew.-% der Salze der Formel (1) aus Glycinbetainlaurylamidsalz bestehen;
(b) 5 bis 20 Gew.-% eines Alkylammoniumsalzes der Formel (2) : $X^{n-}[NH_3^+R]_n$, wobei R eine gesättigte oder ungesättigte lineare Alkylgruppe ist, die 8 bis 18 Kohlenstoffatome umfasst, mit der Maßgabe, dass mindestens 40 Gew.-% der Alkylammoniumsalze der Formel (2) aus Laurylammoniumsalz bestehen;
(c) 5 bis 10 Gew.-% eines Glycinbetainestersalzes der Formel (3): $X^{n-}[(CH_3)_3N^+-CH_2-COOR']_n$, wobei R' ein linearer oder verzweigter Alkylrest ist, der 4 bis 8 Kohlenstoffatome enthält; und
(d) 0 bis 5 Gew.-% Glycinbetain der Formel (4) : $(CH_3)_3N^+-CH_2-COO^-$;

wobei:

X ein organisches oder anorganisches Anion ist und n 1 oder 2 beträgt,
wobei die Verbindungen der Formel (1), (2), (3) und (4) insgesamt 95 bis 100 Gew.-% der Zusammensetzung darstellen.

2. Zusammensetzung nach Anspruch 1, **dadurch gekennzeichnet, dass** die Gruppe X aus Anionen von organischen oder anorganischen Säuren ausgewählt ist, die aus einem Chlorid, einem Sulfat, einem Perchlorat, einem Alkylsulfat-, insbesondere Decylsulfat- oder Laurylsulfation, einem Arylsulfonat-, insbesondere Benzolsulfonat-, Paratoluolsulfonat-, Camphersulfonation, einem Alkylsulfonat-, insbesondere Triflat-, Methansulfonat-, Ethansulfonat-, Decylsulfonat-, Laurylsulfonation, einem Sulfosuccination und Mischungen davon bestehen, wobei X vorzugsweise aus Alkylsulfonaten und Arylsulfonaten, insbesondere aus Methansulfonat-, Ethansulfonat-, Triflat-, Paratoluolsulfonat- und Camphersulfonationen ausgewählt ist, X stärker bevorzugt das Methansulfonation ist.

3. Zusammensetzung nach Anspruch 1 oder 2, **dadurch gekennzeichnet, dass** die Zusammensetzung ausschließlich die Verbindungen der Formeln (1), (2), (3) und (4) umfasst.

4. Verfahren zur Herstellung der Zusammensetzung nach einem der Ansprüche 1 bis 3, das die folgenden aufeinanderfolgenden Schritte umfasst, die aus Folgendem bestehen:

1) dem Umsetzen von Glycinbetain oder einem Salz davon mit einem linearen oder verzweigten $C_4$-$C_8$-Alkohol in Gegenwart einer organischen oder anorganischen Säure bei einer Temperatur von 100 bis 180 °C und unter vermindertem Druck;

2) dem Abkühlen des Reaktionsmediums auf eine Temperatur von 20 bis 80 °C,

3) dem Zugeben eines oder mehrerer Alkylamine, die 8 bis 18 Kohlenstoffatome enthalten, davon mindestens 40 Gew.-% Laurylamin;

4) dem Entfernen des restlichen Alkohols und

5) dem Isolieren der so erhaltenen Tensidzusammensetzung,

**dadurch gekennzeichnet, dass**:

- entweder Schritt (1) unter Bedingungen durchgeführt wird, die den Erhalt eines Umsatzes von Glycinbetain zu Glycinbetainestersalz von mindestens 95 % ermöglichen, gemessen mittels $^1$H-NMR und gemäß der Definition durch die folgende Gleichung:

$$\eta = \frac{I_{XOGBOR'}/2}{I_{XOGB}/2 + I_{XOGBOR'}/2} = \frac{I_{XOGBOR'}}{I_{XOGB} + I_{XOG}}$$

wobei:

- $\eta$ der Umsatz ist
- $I_i$ der Integrationswert des für die Verbindung i charakteristischen Signals [(4,35 ppm, s, 2H) für XOGBOR', (4,28 ppm, s, 2H) für XOGB, wenn X = Mesylat] ist
- XOGBOR' das gebildete Glycinbetainestersalz bezeichnet
- XOGB das gebildete Glycinbetainsalz bezeichnet,

- oder das in Schritt (1) gebildete Glycinbetainestersalz zwischen den Schritten (2) und (3) vom Reaktonsmedium abgetrennt und in Schritt (4) mit dem Alkylamin oder den Alkylaminen umgesetzt wird.

5. Verfahren nach Anspruch 4, **dadurch gekennzeichnet, dass** der Alkohol aus: Butanol, Pentanol, 3-Methylbutan-1-ol (oder Isoamylalkohol), Fuselalkohol (Mischung von Pentanol, 2-Methylbutan-1-ol und 3-Methylbutan-1-ol), Hexanol, Heptanol, Octanol und Mischungen davon, vorzugsweise Butanol, stärker bevorzugt n-Butanol, ausgewählt ist.

6. Verfahren nach Anspruch 4 oder 5, **dadurch gekennzeichnet, dass** 1,1 bis 20 Äquivalente Alkohol, 1,0 bis 1,5 Äquivalente Sulfonsäure und 0,9 bis 1,5 Äquivalente Alkylamin auf 1 Äquivalent Glycinbetain verwendet werden.

7. Verwendung der Zusammensetzung nach einem der Ansprüche 1 bis 3 als Benetzungsmittel, Dispergiermittel für Partikel und/oder Korrosionsinhibitor und/oder zur Verbesserung des Desinfektionsvermögens und/oder der Persistenz der desinfizierenden Wirkung von antimikrobiellen Substanzen und/oder der Wirkung von insektiziden Substanzen.

8. Verwendung der Zusammensetzung nach einem der Ansprüche 1 bis 3 zur Herstellung von Kunststoffen oder von Produkten, die vorgesehen sind:

- zur Behandlung und/oder Reinigung des Körpers, von Pflanzen oder harten Oberflächen, insbesondere von Kosmetika, Produkten zur Fahrzeugreinigung, Haushaltsprodukten, Industriereinigern, Produkten zum Schlichten von Fasern und Pflanzenschutzmitteln;
- zur Wasseraufbereitung;
- zur Erdölgewinnung.

## Claims

1. Surfactant composition comprising:

(a) from 70% to 85% by weight of one or more glycine betaine amide salts of formula (1): $X^{n-}[(CH_3)_3N^+\text{-}CH_2\text{-}CONH\text{-}R]_n$ in which R is a saturated or unsaturated linear alkyl group comprising from 8 to 18 carbon atoms, it being understood that 40% to 100% by weight of the salts of formula (I) consist of glycine betaine laurylamide salt;
(b) from 5% to 20% by weight of alkylammonium salt of formula (2): $X^{n-}[NH_3^+R]_n$ in which R is a saturated or unsaturated linear alkyl group comprising from 8 to 18 carbon atoms, it being understood that at least 40% by weight of the alkylammonium salts of formula (2) consist of laurylammonium salt;
(c) from 5% to 10% by weight of glycine betaine ester salt of formula (3): $X^{n-}[(CH_3)_3N^+-CH_2-COOR']_n$ in which R' is a linear or branched alkyl radical containing from 4 to 8 carbon atoms; and
(d) from 0 to 5% by weight of glycine betaine of formula (4): $(CH_3)_3N^+-CH_2-COO^-$;

in which:

X is an organic or inorganic anion
and n is 1 or 2,
the compounds of formulae (1), (2), (3) and (4) representing in total from 95 to 100% of the weight of the composition.

2. Composition according to Claim 1, **characterized in that** the group X is chosen from anions derived from organic or inorganic acids, consisting of a chloride, a sulfate, a perchlorate, an alkyl sulfate ion, notably decyl sulfate or lauryl sulfate, an arylsulfonate ion, notably benzenesulfonate, para-toluenesulfonate, camphorsulfonate, an alkylsulfonate ion, notably triflate, methanesulfonate, ethanesulfonate, decylsulfonate, laurylsulfonate, a sulfosuccinate ion, and mixtures thereof; preferably, X is chosen from alkylsulfonates and arylsulfonates, in particular from methanesulfonate, ethanesulfonate, triflate, para-toluenesulfonate and camphorsulfonate ions; more preferentially X is the methanesulfonate ion.

3. Composition according to Claim 1 or 2, **characterized in that** the composition exclusively comprises the compounds of formulae (1), (2), (3) and (4).

4. Process for preparing the composition according to any one of Claims 1 to 3, comprising the successive steps consisting in:

1) reacting glycine betaine or a salt thereof with a linear or branched $C_4$-$C_8$ alcohol, in the presence of an organic or inorganic acid, at a temperature of from 100 to 180°C and under reduced pressure;
2) cooling the reaction mixture to a temperature of from 20 to 80°C;
3) adding one or more alkylamines containing from 8 to 18 carbon atoms, including at least 40% by weight of laurylamine;
4) removing the residual alcohol; and
5) recovering the surfactant composition thus obtained,

**characterized in that**:

- either step (1) is performed under conditions making it possible to achieve a degree of conversion of glycine betaine into glycine betaine ester salt of at least 95%, as measured by $^1$H NMR and defined by the following equation:

$$\eta = \frac{I_{XOGBO}/2}{I_{XOGB}/2 + I_{XOGBO}/2} = \frac{I_{XOGBO}}{I_{XOGB} + I_{XOGBOR'}}$$

in which:

• η is the degree of conversion
• $I_i$ is the integration value of the characteristic signal of the compound i [(4.35 ppm, s, 2H) for XOGBOR', (4.28 ppm, s, 2H) for XOGB when X = mesylate]
• XOGBOR' denotes the glycine betaine ester salt formed
• XOGB denotes the glycine betaine salt formed,

- or the glycine betaine ester salt formed in step (1) is separated from the reaction mixture between steps (2) and (3) and reacted with the alkylamine(s) in step (4).

5. Process according to Claim 4, **characterized in that** the alcohol is chosen from: butanol, pentanol, 3-methylbutan-1-ol (or isoamyl alcohol), fusel alcohol (mixture of pentanol, 2-methylbutan-1-ol and 3-methylbutan-1-ol), hexanol, heptanol, octanol, and mixtures thereof, preferably butanol, more preferentially n-butanol.

6. Process according to Claim 4 or 5, **characterized in that** from 1.1 to 20 equivalents of alcohol, from 1.0 to 1.5 equivalents of sulfonic acid and from 0.9 to 1.5 equivalents of alkylamine are used per 1 equivalent of glycine betaine.

7. Use of the composition according to any one of Claims 1 to 3 as a wetting agent, particle dispersant and/or corrosion inhibitor and/or for improving the disinfectant power and/or the persistence of the disinfectant effect of antimicrobial substances and/or the effect of insecticidal substances.

8. Use of the composition according to any one of Claims 1 to 3 for the manufacture of plastics or of products intended:

   - for treating and/or cleansing the body, plants or hard surfaces, in particular cosmetic products, vehicle washing products, household products, industrial cleaning products, fibre sizing products and plant-protection products;
   - for water treatment;
   - for oil extraction.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7829521 B
- WO 2013188508 A
- WO 2017034793 A

**Littérature non-brevet citée dans la description**

- **F. GOURSAUD et al.** *Green Chem,* 2008, vol. 10, 310-320